# FASCICULE DE BREVET EUROPEEN

(11) **EP 0 346 183 B1**
(45) Date de publication et mention de la délivrance du brevet: **21.04.1993**
(21) Numéro de dépôt: 89401468.7
(22) Date de dépôt: 30.05.1989
(51) Int. Cl.: A61F 2/38

(54) **Prothèse à glissement pour le genou**
Gleitprothese für das Knie
Sliding knee prothesis

(30) Priorité: 31.05.1988 FR 8807205
(43) Date de publication de la demande: 13.12.1989
(73) Titulaire: SOCIETE CIVILE D'ETUDE ET DE RECHERCHE DE NOUVELLES PROTHESES (SCERNP), F-75013 Paris (FR)
(72) Inventeur: Judet, Henri, F-75013 Paris (FR); Tourneaud, Jean-Jacques, F-23700 Crocq (FR)
(74) Mandataire: Colas, Jean-Pierre

(56) Documents cités:
- EP-A- 0 135 319
- FR-A- 2 566 657
- GB-A- 2 120 943
- US-A- 4 081 866
- US-A- 4 353 135

## Description

La présente invention concerne une endoprothèse du genou telle que définie dans la première partie de la revendication 1

Dans son certificat d'utilité 84.10261 du 28 Juin 1984 (numéro de publication 2 566 657), la demanderesse a décrit une endoprothèse à glissement pour le genou, comprenant deux pièces solidaires respectivement du tibia et du fémur et une pièce intermédiaire en matière à faible coefficient de frottement, cette pièce et la pièce fémorale pouvant glisser l'une sur l'autre par des surfaces comportant deux fractions qui viennent successivement en appui lors du mouvement de flexion, les rayons de ces fractions étant différents et correspondant aux centres de rotation de l'articulation naturelle. Cette endoprothèse présente par rapport aux prothèses antérieures connues un certain nombre d'avantages importants qui ont été indiqués dans le préambule dudit certificat d'utilité.

Toutefois, le mode de réalisation qui a été décrit présente certains inconvénients qui le rendent peu utilisable en pratique, parce que ne correspondant en fait qu'à des cas limités à des genoux dont les ligaments sont détruits, alors que dans de nombreux cas on peut conserver au moins le ligament croisé postérieur, en plus des ligaments latéraux.

La liaison entre les pièces par axe et galet se déplaçant dans une fente prévue sur l'une des pièces, entraîne un frottement métal sur métal, au moins entre les extrémités de l'axe et les parois de la pièce fémorale entre lesquelles le galet est placé, ce qui est nuisible.

D'autre part, le montage de cette liaison nécessite que l'un des flasques soit démontable, ce qui requiert la présence de vis pour la fixation de ce flasque.

Par ailleurs la présence de l'ouverture oblongue pour le guidage du galet nécessite une grande précision dans l'usinage de cette ouverture ou des ajustages difficiles, afin que les surfaces de glissement de rayons différents soient en appui correct lors du fonctionnement de l'articulation.

L'invention permet d'éviter ces inconvénients et d'obtenir d'autres avantages qui ressortiront de la description suivante.

Dans l'endoprothèse perfectionnée qui fait l'objet de l'invention, les deux parties, solidaires respectivement du fémur et du tibia, et qui sont en contact glissant sur des appuis parallèles, les uns convexes et les autres concaves, conformés avec deux rayons de courbure différents pour réaliser le mouvement en deux temps, sont simplement appliquées l'une contre l'autre par lesdits appuis, sans axe d'articulation matériel. De la sorte, la rotation qui accompagne la flexion du genou est assurée simplement par la coopération des appuis conformés qui peuvent tourner en glissant les uns sur les autres.

Il est vrai que dans le brevet anglais GB-A-2 120 943, sur lequel se fonde le préambule de la revendication 1, a déjà été décrit une prothèse à glissement sans axe matériel entre les pièces fémorales et tibiales qui s'appuient et glissent l'une sur l'autre. Dans cette prothèse la pièce fémorale de forme conique présente de chaque côté du plan de symétrie de l'ensemble, une surface de glissement convexe comportant à l'avant, une première partie à grand rayon de courbure et à l'arrière immédiatement à la suite de la première partie, une seconde partie également convexe mais de rayon de courbure r plus petit, comme dans l'articulation naturelle. La pièce tibiale de forme extérieure conique reçoit une plaquette d'appui emboîtée également conique munie symétriquement de deux pistes de glissement pour les surfaces convexes de la pièce fémorale. Ces deux pistes ont seulement le grand rayon de courbure R, de sorte que dans le repliement du genou, les parties de la pièce fémorale à petit rayon r viennent toucher ponctuellement la pièce tibiale.

Il résulte de ce contact, qui se fait uniquement le long d'une générative des surfaces, que la pression transmise à la pièce tibiale par la pièce fémorale peut devenir très élevée en entraînant une usure rapide des surfaces de glissement.

Des mesures ont en effet montré qu'en raison de la variation des bras de levier, lors d'une rotation du fémur par rapport au tibia par exemple dans le franchissement d'un escalier, la force transmise au tibia par le fémur peut atteindre ou même dépasser le quadruple du poids du corps.

Cet inconvénient est évité dans la prothèse selon l'invention grâce aux particularités exposées dans la revendication 1.

Dans une telle disposition, il est possible de caler angulairement les surfaces d'appui de grand et de petit rayon respectivement, de manière que dans la flexion du genou, les surfaces de petit rayon de la pièce fémorale commencent à épouser les surfaces correspondantes de la pièce tibiale avant que les surfaces correspondantes de la pièce tibiale avant que les surfaces de grand rayon se séparent et ainsi qu'un appui de surface soit maintenu.

Une autre particularité de la prothèse est que celle des parties glissantes qui est en matière à faible coefficient de frottement, telle que du polyéthylène haute densité, et qui est portée par un support métallique solidaire du tibia, est réalisée sous la forme d'une plaquette épaisse dont deux bords opposés portent les appuis concaves conformés aux rayons respectifs et qui est munie de moyens de liaison à la pièce tibiale.

Ladite plaquette est de préférence fixée de façon amovible sur son support métallique, ce qui permet son changement rapide comme son remplacement par une plaquette plus ou moins épaisse pour ajuster la prothése au porteur.

Le dispositif de fixation de la plaquette est avantageusement du genre à liaison par rotation autour d'un axe qui se loge dans la plaquette. Ceci permet de disposer d'une certaine mobilité en rotation verticale du tibia, comme dans l'articulation naturelle.

Au contraire dans le brevet anglais 2 120 943, l'encastrement de la plaquette à contour polygonal dans la pièce tibiale, ne lui permet pas de tourner dans cette pièce, de sorte que la facilité de rotation autour de l'axe du tibia, qui existe dans l'articulation naturelle, fait défaut.

La pièce fémorale, qui est monobloc et dont la forme rappelle celle d'une selle, comporte, sur sa face tournée vers le fémur, des logements où peuvent s'emboîter les condyles du fémur, préalablement conformés et ajustés, ainsi qu'un ou plusieurs appendices destinés à être scellés dans le fémur. Un bon appui entre la pièce et l'os, favorable à une transmission correcte des efforts et à la fixité en rotation, est ainsi assuré.

La pièce tibiale a la forme d'une plaque, munie d'un appendice de scellement dans le canal médullaire du tibia, et assez large pour s'appuyer sur la partie corticale de l'os qui seule a la résistance nécessaire pour supporter le poids du corps. Elle porte en outre un ou deux appendices excentrés qui, en pénétrant dans l'os, assurent sa fixité.

La combinaison de ces moyens procure une prothèse qui, tout en présentant les avantages de la coopération de surfaces glissantes de rayons différents, restituant le mouvement de l'articulation naturelle et n'exigeant pas de résection osseuse importante, est en outre de constitution simple, de réglage facile, mieux adaptée aux désidérata auxquels doit satisfaire une prothèse et permettant dans de nombreux cas de conserver le ligament croisé postérieur en plus des ligaments latéraux.

Dans ce qui suit, on décrira en regard du dessin annexé deux modes de réalisation particuliers de la prothèse perfectionnée, donnés à titre d'exemple non limitatif.

La figure 1 est une vue en perspective de la prothèse dans un premier mode de réalisation convenant à la conservation des ligaments latéraux.

La figure 2 montre également en perspective, mais séparées, les trois parties constitutives de la prothèse.

La figure 3 est une vue de face de la pièce (a).

Les figures 4 et 5 sont des coupes par IV-IV et V-V montrant la prothèse vue de côté, respectivement jambe en extension (figure 4) et en flexion (figure 5).

La figure 6 est une coupe selon VI-VI.

La figure 7 montre en coupe le bord de la plaquette et illustre son dispositif de fixation, sur la pièce tibiale.

Les figures 8 à12 sont relatives à un autre mode de réalisation de la prothèse qui comporte des particularités avantageuses.

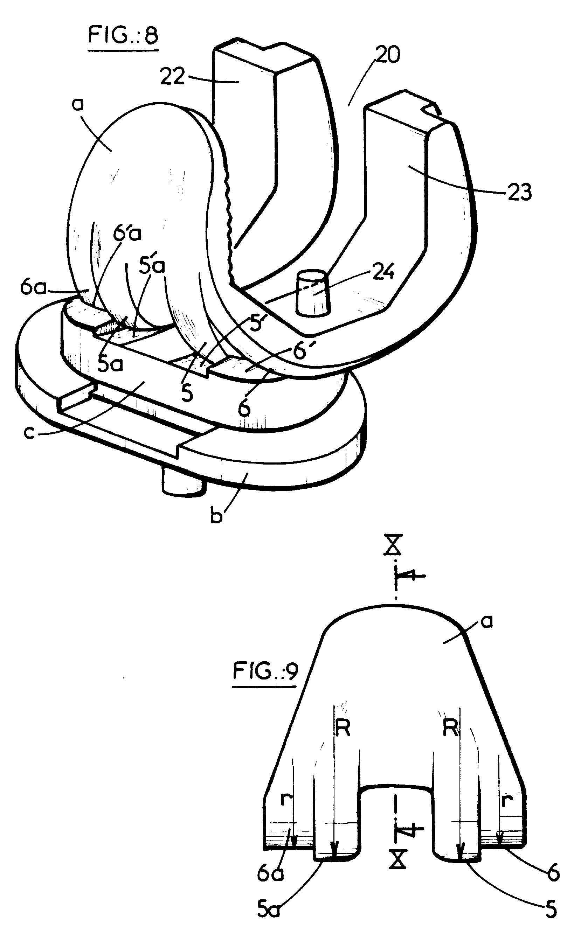

La figure 8 est une vue en perspective de ce mode de réalisation.

La figure 9 est une vue de face de la pièce fémorale.

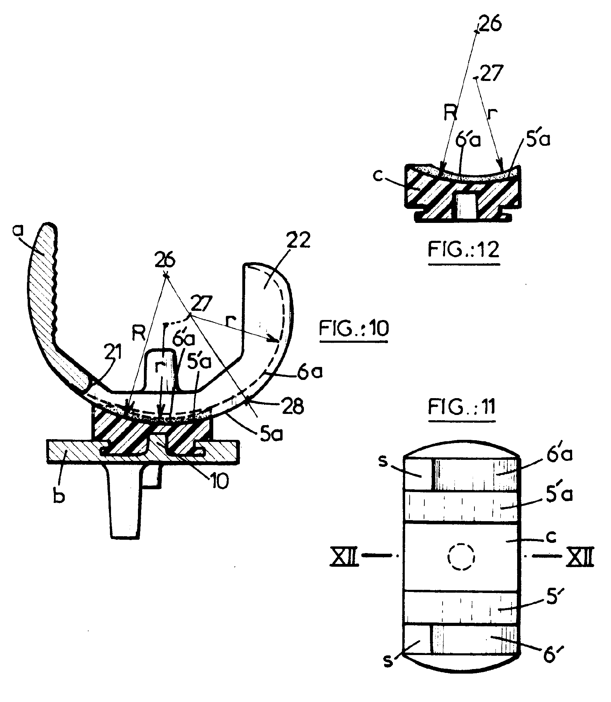

La figure 10 est une coupe selon X-X.

Les figures 11 et 12 montrent une vue en projection horizontale de la plaquette tibiale et une vue en coupe selon XII-XII.

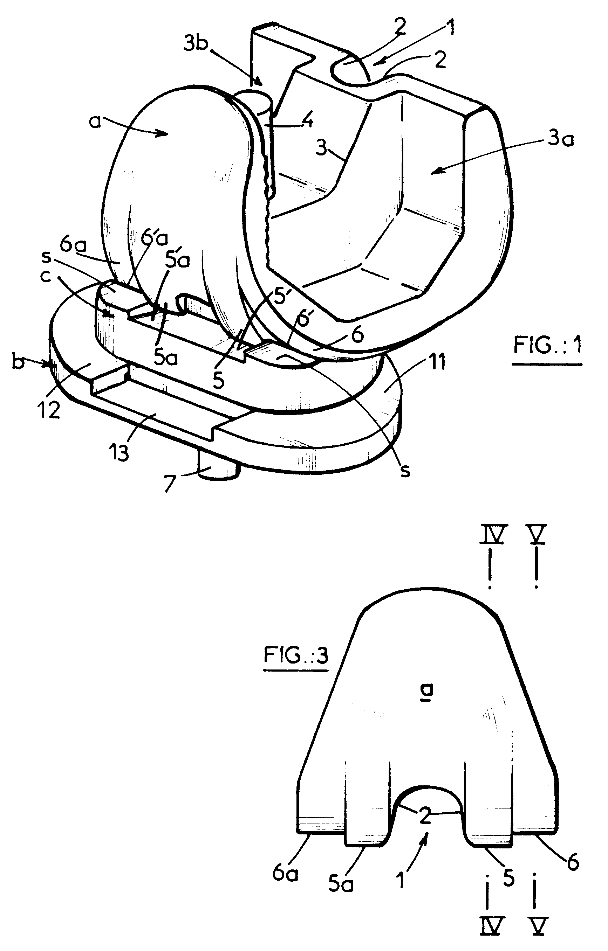

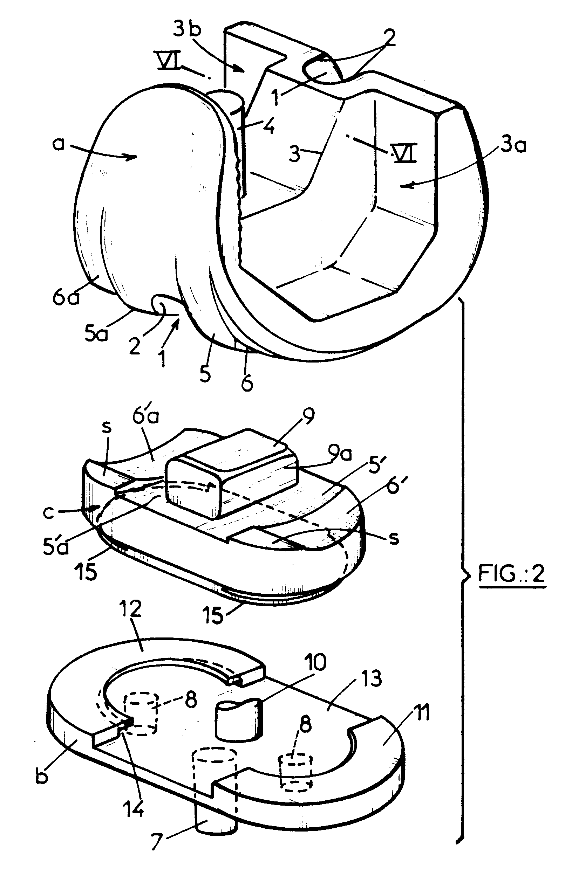

Sur le dessin, a désigne la partie ou pièce de la prothèse qui est solidaire du fémur. Dans le mode de réalisation des figures 1 à 7, une rainure 1, délimitée par deux joues parallèles 2, se poursuit d'avant en arrière en procurant un relief 3 sur le dos de la pièce dont la forme rappelle celle d'une selle. Sur ce relief se trouve un appendice 4 destiné à être scellé dans le canal médullaire du fémur dont les condyles, convenablement préparés et ajustés, viennent se loger à cheval sur le relief 3, dans les cavités telles que 3a et 3b qui existent de part et d'autre de ce relief. On obtient ainsi entre le fémur et la pièce a de la prothèse une liaison favorable à la bonne transmission des efforts et à sa fixité.

La face extérieure de la pièce a comporte les rampes d'appui 5, 6 et 5a, 6a conformées avec des rayons différents.

La pièce b, solidaire du tibia, est constituée par une plaque en métal portant en-dessous un appendice 7 pour le scellement dans le canal médulaire du tibia, ainsi que deux appendices plus petits tels que 8 qui sont introduits dans l'extrémité du tibia convenablement conformée, pour empêcher tout déplacement. La plaque constituant la pièce b a une surface suffisante pour s'appuyer sur la partie corticale du tibia qui est la plus résistante.

Entre les deux parties a et b se trouve une plaquette c de forme oblongue et relativement épaisse, en matière à faible coefficient de frottement, telle que le polyéthylène haute densité. En son centre cette plaquette comporte un bossage 9 venu de fabrication avec la plaquette, et portant un trou cylindrique borgne dans lequel vient s'engager, lors de l'assemblage, un téton cylindrique 10 prévu sur la face supérieure de la plaque tibiale b.

Le bossage 9 comporte deux méplats opposés 9a séparés par une distance qui est égale, au jeu près, à l'intervalle entre les joues 2 de la rainure 1 existant sur la pièce fémorale a.

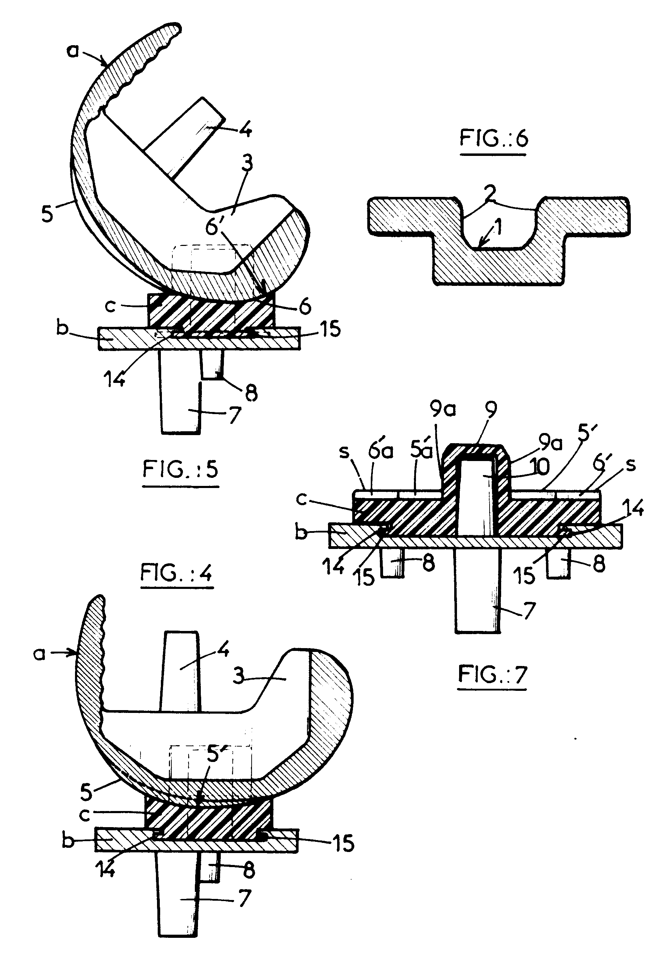

Pour la fixation de la plaquette c sur la pièce tibiale b, celle-ci est évidée sur sa surface supérieure de manière à laisser en relief, à ses extrémités, deux demi-couronnes 11 et 12, séparées par un intervalle 13 très légèrement plus grand que la largeur de la plaquette c. De plus, les demi-couronnes en relief 11, 12 présentent à leur base une rainure semi-circulaire 14, à laquelle correspond sur la face inférieure de la plaquette c une collerette semi-circulaire en saillie 15 (figure 7). Enfin la pièce tibiale b comporte en son centre, sur sa face supérieure, un téton cylindrique 10 qui peut s'engager à frottement doux dans le trou borgne du bossage 10 de la plaquette c.

Pour fixer celle-ci sur la pièce tibiale b, on engage le téton 10 dans le trou du bossage 9, en présentant la plaquette c perpendiculairement à la position qu'elle occupe sur la figure 2, de manière qu'elle repose sur la partie centrale de la pièce b en s'emboîtant dans les intervalles 13 entre les demi-couronnes 11 et 12, puis on fait tourner la plaquette de 90° de manière que les collerettes 15 s'engagent dans les rainures 14, en solidarisant ainsi les pièces b et c dans les position angulaires relatives que montre la figure 2.

On remarque la facilité de ce montage qui offre des possibilités de remplacement rapide de la plaquette c ou pour mettre une plaquette plus ou moins épaisse afin d'ajuster la prothèse à la morphologie du porteur.

On remarque aussi que le montage laisse une faculté de rotation d'un petit angle, sans désassemblage, autour de l'axe du tibia (axe du téton 10) comme dans l'articulation naturelle.

La pièce fémorale en métal a coopère avec la plaquette c en matière à faible coefficient de frottement, liée à la pièce tibiale b, pour permettre le glissement c sur a en même temps que la rotation qui accompagnent la flexion du genou, et ce sans axe matériel d'articulation.

Près de ses petits bords et de part et d'autre du bossage 9, la plaquette comporte deux paires symétriques de chemins de glissement ou rampes concaves 5', 6' ; 5'a, 6'a qui coopèrent avec deux paires symétriques de chemins de glissement ou rampes convexes 5, 6 ; 5a, 6a prévues sur la pièce fémorale a, de part et d'autre de la rainure 1.

Les rampes concaves ainsi que les rampes convexes correspondantes constituent quatre paires associées (5, 5'- 6, 6'- 5a, 5'a et 6a, 6'a) situées dans quatre plans parallèles différentes, comme on le voit bien sur les figures 1, 2, 3, 7 et 11.

Les rampes 5 et 5' ainsi que les rampes coopérantes 5a, 5'a ont le rayon de courbure le plus grand, égal au rayon de la face antérieure des condyles du fémur. C'est le rayon qui a été appelé R dans le certificat d'utilité susmentionné et qui sert à la marche.

Quand la jambe est en extension (figure 4), les rampes 5 et 5a s'appuient sur les rampes 5' et 5'a respectivement et, comme les rayons de courbure sont les mêmes, l'appui se fait sur pratiquement toute la surface des rampes 5' et 5'a de la plaquette, surface qui peut être rendue importante par construction. On peut donc supprimer complètement l'inconvénient des charges ponctuelles.

Quand on fléchit la jambe, le début de la flexion se fait encore par appui des rampes 5 et 5a sur les rampes 5' et 5'a du même rayon, ceci jusqu'à un angle de flexion d'environ 35 à 40°, les rampes 5 et 5a étant suffisamment prolongées sur la pièce fémorale a.

A ce moment, les rampes 6 et 6a de la pièce fémorale a qui ont un rayon de courbure plus petit r et qui ont commencé à être au contact des rampes convexes correspondantes 6', 6'a de même rayon, prévues sur la plaquette, viennent en coïncidence avec ces rampes (figure 5), tandis que les rampes 5 et 5a sont hors d'action. Dans ce cas encore, l'appui des pièces se fait sur une surface importante.

Il est important que le calage angulaire des rampes d'appui de grand et de petit rayon soit tel que les rampes de petit rayon 6 et 6a viennent épouser les rampes correspondantes 6' et 6'a de la plaquette c, avant que les rampes 5 et 5a de grand rayon cessent leur action. On peut ainsi obtenir une continuité dans le mouvement tout en évitant les charges ponctuelles.

De plus, les chemins de glissement 6' et 6'a sont complétés chacun par une saillie s qui s'oppose à une luxation postérieure du genou en remplaçant ou complétant la fonction du ligament croisé postérieur.

Le maintien en appui des pièces de la prothèse, quand le pied n'est pas en appui sur le sol, résulte de l'action des ligaments latéraux de l'articulation naturelle.

Le mode de réalisation qui va maintenant être décrit en regard des figures 8 à 12 a été conçu pour tenir compte de la possibilité avantageuse de conserver le ligament croisé postérieur de l'articulation du genou.

La pièce fémorale a est évidée à cet effet d'une large fente 20 ayant son origine en 21 au bas de la face antérieure de la pièce fémorale. De part et d'autre de cette fente les ailes restantes 22 et 23 sont munies chacune d'un appendice 24 (l'un d'eux est caché sur la figure 8) pour le scellement sur le fémur. Sur leur face tournée vers la plaquette c, les ailes 22 et 23 comportent les surfaces de glissement convexes 5, 6, 5a, 6a coopérant respectivement avec les surfaces concaves 5', 6', 5'a, 6'a de la plaquette.

Comme la fente 20 doit rester libre pour le passage du ligament croisé postérieur de l'articulation naturelle le bossage 9 de la plaquette, ci-dessus décrit, est supprimé, le pivot 10 étant assez court pour se loger dans l'épaisseur de la plaquette. Le guidage de la pièce fémorale sur la plaquette est assuré en augmentant --- la saillie des surfaces glissantes 5, 5a, par rapport aux surfaces 6, 6a et en conformant de façon réciproque les surfaces concaves coopérantes 5', 5'a, 6', 6'a de la plaquette. Grâce à cette mesure on obtient un bon emboitement de la pièce fémorale sur la plaquette, et cet emboîtement assure le guidage.

On voit bien sur les figures 10 à 12 les surfaces convexes à grand rayon (centre 26) de la pièce fémorale qui ont un assez fort relief par rapport aux surfaces de petit rayon dont le centre est en 27 quand la jambe est en extension. Réciproquement les surfaces concaves de petit rayon 6', 6'a de la plaquette c ont un certain relief par rapport aux surfaces concaves de grand rayon 5', 5'a de cette plaquette. Pour illustrer ce relief qui assure le maintien de l'emboitement de guidage, lors de la flexion du genou, on l'a représenté en grisé sur les figures 10 et 12.

28 désigne le joint où s'arrêtent les surfaces à grand rayon 5 et 5a et où commencent les surfaces à petit rayon 6 et 6a.

Les métaux utilisés, côté tibia, comme côté fémur, sont du type acier inoxydable, alliages chrome-cobalt ou titane, tous compatibles avec les tissus. Mais on pourrait sans sortir du cadre de l'invention remplacer tout ou partie des métaux par un matériau dur, compatible avec les tissus.

Il va d'ailleurs de soi que les modes de réalisation décrits pourraient être modifiés, notamment par substitution d'équivalents techniques, sans que l'on sorte pour cela du cadre de l'invention.

## Revendications

1. Endoprothèse du genou dans laquelle deux parties, liées respectivement au fémur et au tibia, et qui sont en contact glissant par des surfaces conformées avec deux rayons de courbure différents (R respectivement r) correspondant aux rayons de courbure de l'articulation naturelle et de même centres que ceux de cette articulation, les mises en coïncidence successives des surfaces coopérantes lors de la flexion du genou, se faisant par un simple guidage de ces surfaces en appui mutuel, sans axes d'articulation matériel, caractérisée en ce que la pièce tibiale (6) comporte deux paires de surfaces d'appui et de glissement (5', 5'a, 6', 6'a) concaves et situées dans des plans différentes symétriquement placés par rapport au plan de symétrie vertical de la prothèse, les surfaces (5', 5'a) de l'une des paires ayant le grand rayon de courbure (R) et les surfaces (6', 6'a) de l'autre paire ayant le petit rayon de courbure (r) tandis que la pièce fémorale (a) comporte deux paires de surfaces d'appui et de glissement convexes (5, 5a, 6, 6a), correspondant dans leur symétrie et leurs rayons aux surfaces respectives de la pièce tibiale.

2. Endoprothèse selon la revendication 1, caractérisée par un calage angulaire des surfaces d'appui de grand et de petit rayon respectivement, tel que dans la flexion du genou les surfaces de petit rayon (6, 6') de la pièce fémorale commencent à épouser les surfaces correspondantes (6a, 6'a) de la pièce tibiale, avant que les surfaces de grand rayon (5, 5', 5a, 5'a) se séparent, de manière à maintenir un appui de surface.

3. Endroprothèse selon l'une des revendications 1 et 2, dans laquelle l'une des parties glissantes (C) est en matière à faible coefficient de frottement, telle que du polyéthylène haute densité, caractérisée en ce que ladite partie est en forme de plaquette épaisse sur laquelle sont évidées symétriquement et parallèlement, les paires de surfaces concaves d'appui conformées (5', 6', 5'a, 6'a) homologues des paires de surfaces convexes (5, 6, 5a, 6a) prévues sur la pièce fémorale (a).

4. Endoprothèse selon la revendication 3, caractérisée en ce que la plaquette (c) est munie d'un bossage central à méplats (9) de petite hauteur, introduit et guidé dans une rainure (1) à bords parallèles de la partie en métal (a) solidaire du fémur qui est monobloc.

5. Endoprothèse selon l'une des revendications 3 et 4, caractérisée en ce que la plaquette (c) est fixée de façon amovible sur un support métallique (b) destiné à être solidarisé du tibia.

6. Endoprothèse selon la revendication 5, caractérisée en ce que le dispositif de fixation de la plaquette est du genre à liaison par rotation autour d'un pivot porté par la pièce tibiale (b) et se logeant dans la plaquette.

7. Endoprothèse selon la revendication 6, caractérisée en ce que le dispositif de fixation de la plaquette est agencé pour permettre en fonctionnement, une petite rotation de la plaquette autour de son pivot.

8. Endoprothèse selon la revendication 6, caractérisée en ce que la pièce tibiale (b) se compose d'un support métallique en forme de plaque, assez large pour s'appuyer sur la partie corticale de l'os et munie vers ses extrémités de deux demi-couronnes en saillie (11, 12) comportant une rainure semi-circulaire (14) dans laquelle vient s'engager par rotation une collerette (15) de la plaquette (c) en matière à faible coefficient de frottement.

9. Endoprothèse selon l'une des revendications 1 à 3 et 5 à 8, caractérisée en ce que le guidage de la pièce tibiale (b et c) par rapport à la pièce fémorale (a) lors de la flexion du genou est assuré par le fait qu'une paire de surfaces cylindriques de glissement (5, 5a) est décalée en hauteur par rapport à l'autre paire (6, 6a) avec un décalage correspondant dans les autres surfaces coopérantes (5', 5'a et 6', 6'a) de manière que le relief ainsi créé assure un emboîtement s'opposant aux mouvements relatifs latéraux des pièces.

10. Endoprothèse selon la revendication 9, caractérisée en ce que la partie postérieure de la pièce fémorale comporte une large fente médiane (20) entre les paires (5-6, 5a-6a) de surfaces coopérantes pour le passage du ligament croisé postérieur de l'articulation naturelle.

## Patentansprüche

1. Knie-Endoprothese, von welcher zwei Teile mit dem Oberschenkelknochen bzw. dem Schienbein verbunden sind und welche in Gleiteingriff durch zueinander konforme Flächen mit zwei unterschiedlichen Kurvenradien (R bzw. r) angeordnet sind, die den Kurvenradien des natürlichen Gelenks entsprechen und die gleichen Mittelpunkte wie die dieses Gelenkes haben, wobei diese in aufeinanderfolgende Koinzidenz der zusammenwirkenden Flächen bei der Beugung des Knies durch eine einfache Führung dieser Flächen in gegenseitiger Abstützung ohne materielle Gelenkachsen realisiert wird, dadurch gekennzeichnet, daß das Schienbeinstück (6) zwei Paare aus Stütz- und Gleitflächen (5', 5'a, 6', 6'a) aufweist, die konkav und in unterschiedlichen zur vertikalen Symmetrieebenen der Prothese symmetrischen Ebenen angeordnet sind, wobei die Oberflächen (5', 5'a) des einen Paares den großen Kurvenradius (R) und die Flächen (6', 6'a) des anderen Paares den kleinen Kurvenradius (r) haben, während der Oberschenkelteil (a) zwei Paare aus Stütz- und Gleitflächen (5, 5a, 6, 6a) aufweist, die konvex ausgebildet sind und in ihrer Symmetrie und hinsichtlich ihrer Radien den entsprechenden Flächen des Schienbeinteils entsprechen.

2. Endoprothese nach Anspruch 1, gekennzeichnet durch eine solche Winkeleinstellung der Stützflächen mit dem großen bzw. dem kleinen Radius, daß beim Beugen des Knies die Flächen mit dem kleinen Radius (6, 6') des Oberschenkelteils beginnen, mit den entsprechenden Flächen (6a, 6') des Schienbeinteils in Eingriff zu treten, bevor die Flächen mit dem großen Radius (5, 5', 5a, 5'a) sich voneinander trennen, wobei eine Flächenabstützung beibehalten wird.

3. Endoprothese nach einem der Ansprüche 1 oder 2, wobei der eine der Gleitteile (c) aus einem Material mit geringem Reibungskoeffizienten gebildet ist, beispielsweise aus Polyethylen mir hoher Dichte, dadurch gekennzeichnet, daß der vorgenannte Gleitteil die Form einer dicken Platte hat, an welcher symmetrisch und parallel zueinander die Paare der konkaven Stützflächen (5', 6', 5'a, 6'a) den Paaren der konvexen Flächen (5, 6, 5a, 6a) an dem Oberschenkelteil (a) entsprechend ausgebildet sind.

4. Endoprothese nach Anspruch 3, dadurch gekennzeichnet, daß die Platte (c) mit einem mittigen Vorsprung mit Abflachungen (9) kleiner Höhe ausgebildet ist, welcher in einer mit parallelen Rändern ausgebildeten Nut (1) des Metallteils (a) geführt wird, welcher seinerseits mit dem Oberschenkelknochen fest verbunden ist.

5. Endoprothese nach einem der Ansprüche 3 oder 4, dadurch gekennzeichnet, daß die Platte (c) auf einem Metallstützteil (b) unbewegbar befestigt ist, der seinerseits mit dem Schienbein fest verbunden wird.

6. Endoprothese nach Anspruch 5, dadurch gekennzeichnet, daß die Befestigungsvorrichtung der Platte in der Art einer Drehverbindung um eine Schwenkachse ausgebildet ist, die durch den Schienbeinteil (b) abgestüzt und in der Platte aufgenommen wird.

7. Endoprothese nach Anspruch 6, dadurch gekennzeichnet, daß die Befestigungsvorrichtung der Platte eingerichtet ist, um in der Funktion eine kleine Drehbewegung der Platte um ihre Schwenkachse zuzulassen.

8. Endoprothese nach Anspruch 6, dadurch gekennzeichnet, daß der Schienbeinteil (b) aus einem Metallstützteil in der Form einer Platte besteht, der ausreichend groß ist, um sich auf dem kortikalen Teil des Knochens abzustützen und der an seinen äußeren Bereichen mit zwei vorspringenden Halbkronenteilen (11, 12) versehen ist, die jeweils eine Halbkreisnut (14) aufweisen, in welchen ein Flansch (15) der Platte (c) aus einem Material mit einem geringen Reibungskoeffizienten drehbar gehaltert ist.

9. Endoprothese nach einem der Ansprüche 1 bis 3 und 5 bis 8, dadurch gekennzeichnet, daß die Führung des Schienbeinteils (b und c) im Hinblick auf den Oberschenkelteil (a) bei der Beugung des Knies durch die Tatsache sichergestellt ist, daß ein Paar der zylindrischen Gleitflächen (5, 5a) in der Höhe im Hinblick auf das andere Paar (6, 6a) in einer den anderen zusammenwirkenden Flächen (5', 5'a und 6', 6'a) entsprechenden Einstellung so ausgerichtet ist, daß das so gebildete Relief eine Fügung sicherstellt, die sich seitlichen Relativbewegungen der Teile widersetzt.

10. Endoprothese nach Anspruch 9, dadurch gekennzeichnet, daß der hintere Teil des Oberschenkelteils zwischen den Paaren (5-6, 5a-6a) der zusammenwirkenden Flächen für den Durchgang des hinteren Kreuzbandes des natürlichen Gelenkes einen großen mittleren Spalt (20) aufweist.

## Claims

1. Endoprosthesis for the knee in which two parts, respectively linked to the femur and to the tibia, and which are in sliding contact via shaped surfaces with two different radii of curvature (respectively R, r) corresponding to the radii of curvature of the natural joint and with centres the same as those of this Joint, the successive setting of the interacting surfaces in coincidence during the bending of the knee being made by simple guiding of these mutually bearing surfaces, without a physical articulation axis, characterised in that the tibial piece (6) includes two pairs of bearing and sliding surfaces (5', 5'a, 6', 6'a) which are concave and situated in different planes which are symmetrically placed with respect to the vertical plane of symmetry of the prosthesis, the surfaces (5', 5'a) of one of the pairs having the large radius of curvature (R) and the surfaces (6', 6'a) of the other pair having the small radius of curvature (r) while the femoral piece (a) includes two pairs of convex bearing and sliding surfaces (5, 5a, 6, 6a), corresponding in their symmetry and their radii to the respective surfaces of the tibial piece.

2. Endoprosthesis according to Claim 1, characterised by angular setting of the bearing surfaces of large and small radius respectively, such that on bending of the knee, the surfaces of small radius (6, 6') of the femoral piece start to match the corresponding surfaces (6a, 6'a) of the tibial piece, before the surfaces of large radius (5, 5', 5a, 5'a) separate, so as to maintain surface bearing.

3. Endoprosthesis according to either of Claims 1 or 2, in which one of the sliding parts (C) is made of a material with a low coefficient of friction, such as high-density polyethylene, characterised in that the said part is in the form of a thick block on which there are hollowed in a symmetrical parallel manner, the pairs of shaped concave bearing surfaces (5', 6', 5'a, 6'a) homologous with the pairs of convex surfaces (5, 6, 5a, 6a) provided on the femoral piece (a).

4. Endoprosthesis according to Claim 3, characterised in that the block (c) is provided with a central boss with flats (9) of small height, introduced and guided in a groove (1) with parallel edges of the metal part (a) solidly attached to the femur which is monobloc.

5. Endoprosthesis according to either of Claims 3 or 4, characterised in that the block (c) is fixed removaly onto a metallic support (b) intended to be solidly attached to the tibia.

6. Endoprosthesis according to Claim 5, characterised in that the fixing device of the block is of the type with linkage by rotation around a pivot carried by the tibial piece (b) and being housed in the block.

7. Endoprosthesis according to Claim 6, characterised in that the fixing device of the block is arranged in order to allow during operation, a small rotation of the block around its pivot.

8. Endoprosthesis according to Claim 6, characterised in that the tibial piece (b) is composed of a metallic support in plate form, which is wide enough to bear on the cortical part of the bone and provided towards its ends with two projecting half-rings (11, 12) including a semicircular groove (14) in which a collar (15) of the block (c) made of a material with a low coefficient of friction is engaged by rotation.

9. Endoprosthesis according to one of Claims 1 to 3 and 5 to 8, characterised in that the guiding of the tibial piece (b and c) with respect to the femoral piece (a) during the bending of the knee is ensured by the fact that one pair of cylindrical sliding surfaces (5, 5a) is offset in height with respect to the other pair (6, 6a) with a corresponding offset in the other interacting surfaces (5', 5'a and 6', 6'a) such that the profile thus created ensures fitting which opposes the relative lateral movements of the pieces.

10. Endoprosthesis according to Claim 9, characterised in that the posterior part of the femoral piece includes a wide median slot (20) between the pairs (5-6, 5a-6a) of interacting surfaces for the passage of the posterior cruciate ligament of the natural joint.
